(19) Europäisches Patentamt
European Patent Office
Office européen des brevets

(11) Veröffentlichungsnummer: **0 489 776 B1**

(12) # EUROPÄISCHE PATENTSCHRIFT

(45) Veröffentlichungstag der Patentschrift: **25.10.95**

(51) Int. Cl.6: **A61L 2/20**, C01B 11/02

(21) Anmeldenummer: **90912664.1**

(22) Anmeldetag: **21.08.90**

(86) Internationale Anmeldenummer:
**PCT/EP90/01377**

(87) Internationale Veröffentlichungsnummer:
**WO 91/03265 (21.03.91 91/07)**

(54) **VERFAHREN ZUR DESINFEKTION VON HARTEN OBERFLÄCHEN MIT CHLORDIOXID.**

(30) Priorität: **30.08.89 DE 3928747**

(43) Veröffentlichungstag der Anmeldung:
**17.06.92 Patentblatt 92/25**

(45) Bekanntmachung des Hinweises auf die Patenterteilung:
**25.10.95 Patentblatt 95/43**

(84) Benannte Vertragsstaaten:
**AT BE CH DE DK ES FR GB IT LI LU NL SE**

(56) Entgegenhaltungen:
**WO-A-84/01507**
**WO-A-85/04107**
**DE-A- 2 903 980**
**US-A- 4 504 442**
**US-A- 4 681 739**

**Chemical Abstracts, Vol. 105, No. 8, 25 August 1986, (Columbus, Ohio US), page 84, abstract 62156a.**

**Dialog Information Services; File 351, World Patent Index 81-90, Dialog accession no. C89-105613, Diacel Chem Ind KK: "Sterilisation of the soil - by covering surface with non-permeable sheet and blowing air contg. chlorine dioxide between soil, and sheet".**

(73) Patentinhaber: **Henkel Kommanditgesellschaft auf Aktien**

**D-40191 Düsseldorf (DE)**

(72) Erfinder: **SCHRÖDER, Karl-Heinz**
**Hermann-von-Endt-Strasse 3**
**D-4000 Düsseldorf 13 (DE)**
Erfinder: **FALTER, Wolfgang**
**Todtleger 23**
**B-4731 Lichtenbosch (BE)**
Erfinder: **GROSSE-BÖWING, Walter**
**Melanderstrasse 23**
**D-4047 Dormagen 5 (DE)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 489 776 B1

## Beschreibung

Die Erfindung betrifft ein Verfahren zur Desinfektion von harten Oberflächen unter Wasserhärte-stabilisierten und nicht korrosiven Bedingungen mit wäßrigen Lösungen von Chlordioxid, hergestelllt in Lösung durch Mischen einer Lösung aus Natriumchlorit und einer sauren Komponente in einem Generator.

Chlordioxid ist ein Desinfektionsmittel, welches im Vergleich zu Chlor, Alkali- bzw. Erdalkalihypochloriten, organischen Chlorträgern und anderen Desinfektionsmitteln, die auf Aktivchlorbasis beruhen, vor allem bei der Desinfektion von Flaschen, Behältern, Geräten sowie Leitungen Vorteile aufweist. Chlordioxid bildet praktisch keine Trihalomethane (Haloforme) sowie kaum höher-molekulare Organohalogenverbindungen. Im Gegensatz zu Chlor reagiert Chlordioxid nicht mit Phenolen zu Chlorphenolen, die für den Apothekergeschmack bei der Reinigung von Mineral- und Tafelwasserflaschen verantwortlich sind. Desgleichen bleibt eine Reaktion von Chlordioxid mit Amino- oder Ammoniumverbindungen zu Chloraminen oder Ammoniumchloriden aus. Die Wirksamkeit (Redoxpotential) von Chlordioxid, die zudem nahezu pH-unabhängig ist, ist zwei- bis dreimal stärker als die von Chlor. Noch deutlicher wird dies jedoch im Vergleich mit Persäuren, die ebenfalls zur Desinfektion eingesetzt werden. Neben höherer Effektivität resultiert hieraus die Anwendung geringerer Konzentrationen und die damit verbundene geringere Abwasserbelastung.

Neben einer bakteriziden Wirkung zeigt Chlordioxid auch gute sporizide, viruzide und algizide Eigenschaften. Chlordioxid ist ein Desinfektionsmittel mit hoher Aktivität und verleiht gleichzeitig einen langanhaltenden bakteriziden und bakteriostatischen Schutz. Neben dem Einsatz als Desinfektionsmittel bewährt sich Chlordioxid aufgrund seines hohen Oxidationspotentials auch bei der Wasseraufbereitung zur Beseitigung unangenehmer Gerüche, Geschmäcker und Farben. Es reagiert zudem mit organisch gebundenem Eisen und Mangen (z.B. in Humin- und Fulvinsäuren) und baut cancerogene Substanzen wie polycyclische Aromaten ab. Bei der Vorbehandlung von Roh- bzw. Oberflächenwässern wird die Flockung verbessert, weswegen Chlordioxid bevorzugt zur Trinkwasserdesinfektion eingesetzt wird. Ein weiteres Anwendungsgebiet der Desinfektionswirkung von Chlordioxid ist in der Lebensmittel- und Getränkeindustrie, z.B. bei der Desinfektion im Bereich des Füllers, in Flaschenwaschmaschinen und Pasteuren, als Vor- und Rücklaufwasser sowie Wasch- und Spülwasser. Problematisch sind hier vor allem Ausfällungen insbesondere von Wasserhärte, die insbesondere bei pH-Werten zwischen 10,5 und 11,5 und/oder bei Temperaturen über 54 °C einsetzen. Aber auch bei niedrigeren und höheren pH-Werten und/oder Temperaturen treten unerwünschte Ausfällungen von Wasserinhaltsstoffen, insbesondere der Wasserhärte, auf.

Bei dem derzeitigen Verfahren zur Herstellung von Chlordioxid wird von dem Chlor-Chlorit- (1) bzw. dem Salzsäure-Chlorit-Verfahren (2) ausgegangen. Das Chlor-Chlorit-Verfahren wird bevorzugt bei der kommunalen Wasseraufbereitung (Großanwender), das Salzsäure-Chlorit-Verfahrenin Industriebetrieben (Klein-, Mittelanwender) eingesetzt.

$$2\ NaClO_2 + Cl_2 \longrightarrow 2\ ClO_2 + 2\ NaCl \quad (1)$$

$$5\ NaClO_2 + 4\ HCl \longrightarrow 4\ ClO_2 + 5\ NaCl + 2\ H_2O \quad (2)$$

$$2\ NaClO_2 + Cl_2 \xrightarrow[\text{pH-Einstellung}]{\text{Säure}} 2\ ClO_2 + 2\ NaCl \quad (3)$$

Beim Chlor-Chlorit-Verfahren (1) ist, um eine vollständige Umsetzung gemäß der Reaktionsgleichung zu erzielen, ein bestimmter Überschuß an Chlor einzusetzen, was dazu führt, daß bei der Anwendung immer überschüssiges Chlor anwesend ist. Bei stöchiometrischem Einsatz erfolgt nur eine unzureichende Umsetzung.

Beim Salzsäure-Chlorit-Verfahren (2) werden wenig verunreinigte Chlordioxid-Lösungen erhalten, allerdings ist die maximale theoretische, auf Chlorit bezogene Ausbeute aufgrund der Stöchiometrie der Reaktionsgleichung auf 80 % beschränkt. Neben Salzsäure können hier auch andere Säuren eingesetzt werden.

Das Dreikomponentenverfahren (3) basiert auf dem Chlor-Chlorit-Verfahren (1) und gewährleistet durch den Einsatz zusätzlicher Säure einen praktisch vollständigen Chlorit-Umsatz, bedingt durch die pH-Wert-Absenkung.

Nachteilig bei den derzeitig technisch eingesetzten Verfahren ist die Korrosionswirkung, da Salzsäure im derzeitigen Salzsäure-Chlorit-Verfahren (2) in großem Überschuß eingesetzt wird. Salzsäure bzw. Chlor tragen bekanntermaßen besonders zur Lochfraßkorrosion, insbesondere an Edelstählen bei. Darüber hinaus sind die z.T. hohen Kochsalzkonzentrationen, die bei diesen Verfahren freigesetzt werden, ebenfalls von Nachteil. Die Verfahren zur Des infektion bleiben daher auf bestimmte Anwendungen beschränkt.

Das Ziel von WO 85/04107 ist, die aggressive Salzsäure durch $\alpha$-Hydroxycarbonsäuren zu ersetzten. In JP 86/145 300 werden zur Herstellung von Chlordioxid Essig-, Schwefel-, Citronen- oder Malonsäure eingesetzt. Das so hergestellte Chlordioxid dient zur Desinfektion von Fischnetzen. JP 76/082 080 verwendet ebenfalls Citronensäure zum Herstellen von Bleichbädern. Zum Bleichen von Fasern wird nach JP 85/259 671 eine Mischung von Natriumchlorit bzw. chloriger Säure und organischen Phosphonsäuren benutzt. Aus verschiedenen Säuren generiertes Chlordioxid kann gemäß WO 84/01507 zur Sterilisation von medizinischen Geräten verwendet werden. JP 84/187 668 beschreibt die Freisetzung von Chlordioxid mit Hilfe von Tetrachlorethylen in Sauerstoff, US 4,542,008 und US 4 432 856 beschreiben die elektrolytische Zersetzung von Natriumchlorit zu Chlordioxid.

Gegenüber der Herstellung von Chlordioxid aus Natriumchlorit und Säure sind Verfahren bekannt, gemäß welchen Chlordioxid hergestellt wird aus Chlorat und Chlorsäure, mit Hilfe von unterschiedlichen Reduktionsmitteln wie Wasserstoffperoxid in JP 88/008 203, Chlorid und Säure in EP 153 841, Chlorid und Hitze in US 4 678 653, Schwefeldioxid und Säure in WO 83/01940, Salzsäure in DE 32 18 649, Alkoholen und Säure in US 4 627 969, Mangan (II)-Salzen in JP 85/054 923, Hypochlorit und Säure in JP 83/161 903 und Palladium-(II)-Salzen in US 4 421 730.

Dennoch sind keine Verfahren bekannt, die es ermöglichen, die hohe Korrosivität von Chlordioxid bei seinen Anwendungen zu kontrollieren. Neben diesen ungelösten Korrosionsproblemen verhindern auch Härteausfällungen beim Einsatz in Hartwasser sowie die hohe Salzbelastung des Abwassers einen großflächigen Einsatz von Chordioxid. Durch diese Nachteile bleiben zudem weitere Anwendungsbereiche dem Chlordioxid verschlossen.

Der Erfindung liegt daher die Aufgabe zugrunde, ein Verfahren zur Desinfektion von harten Oberflächen unter Verwendung von Chlordioxid zu entwickeln, bei dem gleichzeitig die Korrosion verringert und die Ausfällungen, insbesondere der Wasserhärte verhindert werden. Darüber hinaus sollte die Kochsalzbelastung der Abwässer verringert werden.

Die Aufgabe wurde erfindungsgemäß gelöst durch ein Verfahren zur Desinfektion von harten Oberflächen unter Wasserhärte-stabilisierten und nicht korrosiven Bedingungen mit wäßrigen Lösungen von Chlordioxid, hergestellt in Lösung durch Mischen einer Lösung aus Natriumchlorit und einer sauren Komponente in einem Generator,
dadurch gekennzeichnet, daß

a) die Konzentration der Natriumchlorit-Lösung 2 bis 40 Gew.-% beträgt,

b) als saure Komponente, Wasserhärtestabilisator und Korrosions inhibitor ein stickstofffreies Sequestrierungsmittel oder Komplexierungsmittel, ausgewählt aus Phosphonsäuren, Polyphosphorsäure, Acrylsäure, Methacrylsäure, Polyacrylsäure oder Polymethacrylsäure, oder Gemische derartiger Sequestrierungsmittel und Komplexierungsmittel verwendet werden,

c) wobei das Sequestrierungsmittel und/oder Komplexierungsmittel, dessen wäßrige 1 %-ige Lösung einen pH-Wert von < 7 aufweist, in einer Konzentration von 1 bis 60 Gew.-% eingesetzt wird,

d) zusätzlich zu den Sequestrierungsmitteln und/oder Komplexierungsmitteln Carbonsäuren und/oder Hydroxycarbonsäuren, ausgewählt aus Weinsäure, Äpfelsäure, Oxalsäure, Maleinsäure, Malonsäure, Bernsteinsäure, Adipinsäure, Glycolsäure, Milchsäure, Gluconsäure oder Citronensäure, eingesetzt werden,

e) wobei das Mischungsverhältnis (bezogen auf Molverhältnisse) von Sequestrierungs- und/oder Komplexierungsmittel zu Carbonsäure oder Hydroxycarbonsäure im Bereich von 10 : 1 bis 1 : 10 liegt,

f) und die Chlordioxid-Konzentration zwischen 0,1 und 500 ppm beträgt.

Komplexierungs- und Sequestrierungsmittel, die für den genannten Einsatzzweck geeignet sind, kann der Fachmann auch daran erkennen, daß sie in einem oder mehreren der Tests A, B, C (Warmwasserzonensimulator, Belagsverhalten im Wechseltaucher und Calciumbindevermögen) eine bessere Belagsinhibierung zeigen als der entsprechende Wasserwert.

Bevorzugt weist das Sequestrierungsmittel oder das Komplexierungsmittel als wäßrige 1 %-ige Lösung einen pH-Wert von < 3 auf, und die Chlordioxid-Konzentration beträgt bevorzugt 0,1 bis 100 ppm.

Im Sinne des erfindungsgemäßen Verfahrens sind Chlordioxid-Konzentrationen im Bereich von 0,1 bis 20 ppm und insbesondere im Bereich von 0,5 bis 5 ppm besonders bevorzugt.

Die Konzentration des Sequestrierungsmittels und/oder Komplexierungsmittels liegt vorzugsweise im Bereich von 15 bis 25 Gew.-%.

Als Sequestrierungsmittel oder Komplexierungsmittel werden eingesetzt: Phosphonsäuren, Polyphosphorsäure, Acrylsäure, Methacrylsäure, Polyacrylsäure sowie Polymethacrylsäure. Besonders bevorzugt eingesetzte Phosphonsäuren, deren wäßrige 1 %-ige Lösung einen pH-Wert von < 7, vorzugsweise < 3, aufweist, und die einen oder mehrere der Tests A, B oder C erfüllen, sind 1-Hydroxyethan-1,1-diphosphonsäure und 2-Phosphonobutan-1,2,4-tricarbonsäure.

Als Beispiele für weitere Phosphonsäuren, die im Sinne der Erfindung Verwendung finden können, sind zu nennen: 2,2-Diphosphonobutan-3,4-dicarbonsäure, 1-Phosphonopropan-1,2,3-tricarbonsäure, 1,1-Diphosphonopropan-2,3-dicarbonsäure und Methylendiphosphonsäure.

Demgegenüber setzen N-haltige Sequestrierungs- und Komplexierungsmittel das Chlordioxid nicht immer in der für das erfindungsgemäße Verfahren gewünschten Art und Weise frei.

Bei dem erfindungsgemäßen Verfahren zur Desinfektion von harten Oberflächen werden als saure Komponenten Gemische der genannten Sequestrierungs- und/oder Komplexierungsmittel mit Carbonsäuren und/oder Hydroxycarbonsäuren, die eine oder mehrere Carboxylgruppen aufweisen, eingesetzt werden. Als Carbonsäuren und/oder Hydroxycarbonsäuren, die hierbei in Frage kommen, seien genannt: Citronensäure, Weinsäure, Äpfelsäure, Oxalsäure, Maleinsäure, Malonsäure, Bernsteinsäure, Adipinsäure, Glycolsäure, Milchsäure oder Gluconsäure. Demgemäß ist es im Sinne des erfindungsgemäßen Verfahrens bevorzugt, 2-Phosphonobutan-1,2,4-tricarbonsäure und/oder 1-Hydroxyethan-1,1-diphosphonsäure zusammen mit Citronensäure einzusetzen. Anstelle von Citronensäure kann hierbei insbesondere auch Milchsäure Verwendung finden. Das Mischungsverhältnis (bezogen auf Molverhältnisse) von Sequestrierungs- und/oder Komplexierungsmittel zu Carbonsäure oder Hydroxycarbonsäure liegt im Bereich von 10 : 1 bis 1 : 10, wobei Mischungsverhältnisse im Bereich von 2 : 1 bis 1 : 2 und insbesondere von 1 : 1 besonders bevorzugt sind. Derartige Zusätze bedingen im Rahmen des erfindungsgemäßen Verfahrens in synergistischer Weise eine Steigerung der Ausbeute an Chlordioxid sowie eine bessere Belagsinhibierung.

Gemäß einer weiteren Ausführungsform der vorliegenden Erfindung können zusätzlich zu der vorstehend beschriebenen Kombination von Sequestrierungsmitteln mit Carbonsäuren oder Hydroxycarbonsäuren noch Lewissäuren als saure Komponenten eingesetzt werden. Als bevorzugte Lewissäuren sind hier Eisen- und/oder Aluminiumsalze, insbesondere die entsprechenden Sulfate, Nitrate oder Chloride, zu nennen. Die Menge derartiger Zusätze ist beliebig und kann in breiten Grenzen varriert werden. Bevorzugt hierbei sind Mischungsverhältnisse (bezogen auf Molverhältnisse) von Sequestrierungsmitteln und Carbonsäuren oder Hydroxycarbonsäuren zu Lewissäuren im Bereich von 1 : 2 bis 3 : 1.

Die Reaktionsweise wird durch nachfolgendes allgemeines Formelschema gekennzeichnet:

$$5\ NaClO_2 + 4/n\ (R - XH_n)_m \text{--->} 4\ ClO_2 + 4/n\ ((RX_n)_m)^{n-}\ nNa^+ + 2\ H_2O + NaCl$$

Charakteristisch für die Sequestrierungsmittel ist, daß sie in weit weniger als äquimolaren Mengen, bezogen auf das Kation, dessen Kristallaufbau verhindert werden soll, eingesetzt werden. Dies bedeutet beispielsweise, daß für 1 °d (in 1 % NaOH) weniger als 100 ppm, bevorzugt weniger als 50 ppm, Aktivsubstanz benötigt werden. Komplexierungsmittel hingegen werden in einer Konzentration eingesetzt, die gleich oder höher ist, als zur stöchiometrischen Wasserhärtestabilisierung notwendig.

Für die erfindungsgemäßen Chlordioxid/Korrosions inhibitor/Härtestabilisator-Lösungen, die durch dieses Einstufen-Verfahren aus lediglich drei Komponenten generiert werden können, ergeben sich weitere Anwendungsbereiche, wie insbesondere die Sprüh- und Schaumaußenreinigung von Anlagen, Desinfektion von Trinkwasserkreisläufen, Membrananlagen, Tanks, Behältern und Füllern. Ein Einsatz kann ebenfalls als Additiv zu Kettengleitmitteln als Reinigungs- und vor allem als Desinfektionskomponente erfolgen.

Da die im Sinne des erfindungsgemäßen Verfahrens erzeugten Lösungen die Wirkung eines Desinfektionsmittels, eines Wasserhärtestabilisators und eines Korrosions inhibitors in sich vereinigen, können sie insbesondere in Flaschenwaschmaschinen, Tunnelpasteuren, Rückkühlern, Filteranlagen sowie zur Kühlwasserdesinfektion, Faß- und Containerreinigung angewendet werden. Hierzu waren bisher neben Chlordioxid noch weitere zusätzliche Komponenten als Härtestabilisator und Korrosions inhibitor notwendig, die den bekannten Lösungen zudosiert werden mußten. Der Einsatz eines zusätzlichen Härtestabilisators und Korrosions inhibitors kann nach dem erfindungsgemäßen Verfahren unterbleiben. Die wahlweise Zudosage eines Reinigungsmittels, Reinigungsverstärkers, oberflächenaktiver Verbindungen und anderer für den Anwendungszweck üblichen Zusatz- und Hilfsstoffe, stört die Durchführung und Anwendung des erfindungsgemäßen Verfahrens im allgemeinen nicht.

Die Vorteile seien am Beispiel der bevorzugt eingesetzten Phosphonsäuren, z.B. der 2-Phosphonobutan-1,2,4-tricarbonsäure erläutert: Die als Sekundärprodukte anfallenden Alkalimetallphosphonate bzw. noch in zusätzlichem Überschuß vorhandene freie Phosphonsäure stabilisieren die Wasserhärte, d.h. Calcium- und Magnesiumionen. Dies ist auch dann der Fall, wenn die Sequestrierungs- und Komplexierungsmittel in

einer alkalischen Gebrauchslösung als entsprechende Alkalimetallsalze vorliegen. Der Einsatz von zusätzlichen Komplexierungs- und Sequestrierungsmitteln kann entfallen. Durch den Verzicht auf aggressive anorganische Säuren wird ein korrosiver Angriff auf Messing, Kupfer, Edelstahl und Aluminium vermindert. Die Einsparung von zwei weiteren Komponenten macht das erfindungsgemäße Verfahren kostengünstig und verbessert die Arbeitssicherheit bei der Durchführung am Ort der Anwendung.

Darüber hinaus ist die Wirksamkeit zur Desinfektion, Härtestabilisierung und Korrosionsinhibierung durch die Verwendung eines beliebigen Überschusses an saurer Komponente frei steuerbar, d.h. für besonders hartes Wasser kann entsprechend mehr als nur die äquimolare Menge an Sequestrierungs- oder Komplexierungsmittel und den Carbonsäuren und/oder Hydroxycarbonsäuren zugesetzt werden, ohne gleichzeitig die Desinfektionswirkung oder Korrosionsinhibierung zu verringern.

Die Tests A, B und C zur Prüfung der Komplexierungs- und Sequestrierungseigenschaften werden nachfolgend für spezielle Verbindungen beispielhaft erläutert.

Test A:

Das belagsverhindernde Verhalten der erfindungsgemäßen Desinfektions lösungen wurde in einer Warmwasserzone (50 ± 0,5 °C) geprüft, in die ein gereinigtes und gewogenes Chrom-Nickel-Stahl-Blech (V 4A) bekannter Oberfläche eingebracht war. Der Wasserdurchfluß von Betriebswasser mit 16 °d betrug 25 l/h, die Versuchsdauer 6 h bei verschiedenen pH-Werten von 9, 10, 11 und 12. Anschließend wurde das Testblech entfernt und gewogen. Bei einem pH von 10 und einem Sequestriermittelzusatz von 30 $g/m^2/h$ betrug die Belagbildung weniger als 0,2 $g/m^2/h$. Bei einem pH von 11, einem Sequestriermittelzusatz von 50 $g/m^2/h$ betrug die Belagbildung weniger als 0,5 $g/m^2/h$.

Als Sequestriermittel wurde hierbei ein Gemisch aus 2-Phosphonobutan-1,2,4-tricarbonsäure und Citronensäure - Molverhältnis 1 : 1 - eingesetzt.

Test B:

Gleichermaßen kann das Belagsverhalten durch einen Wechseltauchtest bestimmt werden, bei dem ein Testblech oder Glas je 2 min in eine Lauge, die auch die Desinfektionsmittellösung enthält, ein Spülbad (Wasser) getaucht und anschließend getrocknet wird.

Test C:

Die Bestimmung des Calcium- und Magnesiumbindevermögens (CaBv und MgBv) erfolgt wie nachfolgend beschrieben:

Nach Bestimmung der Glührückstands-Werte, wird in Abhängigkeit derer die ungefähre Einwaage an Aktivsubstanz (As) ermittelt. Die Menge an Aktivsubstanz sollte ca. 200 mg betragen. Die auf der Analysenwaage eingewogenen Mengen werden dann in einen 300 ml Erlenmeyerkolben gegeben und mit 200 ml $H_2O$ 30 °d oder 6 °d versetzt und 10 min gerührt. Danach werden die Proben über eine Nutsche oder Fritte abgesaugt. 100 ml des Filtrats werden abpipettiert und mit 1 Indikator-Puffertablette (Ammoniumchlorid, Firma Merck, Artikel-Nr. 8430) versetzt. Wenn sich diese gelöst hat, werden noch 5 ml konzentrierte Ammoniak-Lösung hinzugegeben. Anschließend wird mit n/28-Komplexon$^R$-Lösung (Farbumschlag rot-grün) titriert und der Verbrauch (V) ermittelt. Ein Blindwert (Bw) ohne Aktivsubstanzeinwaage wird unter gleichen Bedingungen durchgeführt.

1 °d = 10 mg CaO/l bzw. MgO/l x F (0,719)

Berechnung:

für Wasser mit 6 °d (Deutsche Härte):

CaBv MgBv

$$CaBv = \frac{(Bw-V) \cdot 2}{As(in\ g) \cdot 5} \quad ; \quad MgBv = \frac{(Bw-V) \cdot 2 \cdot 0,719}{As\ (in\ g) \cdot 5}$$

für Wasser mit 30 °d (Deutsche Härte):

$$CaBv = \frac{(Bw-V) \cdot 2}{As(in\ g)} \quad ; \quad MgBv = \frac{(Bw-V) \cdot 2 \cdot 0,719}{As\ (in\ g)}$$

Das Ergebnis wird angegeben in: mg CaO/g As bzw. mg MgO/g As

Test D:

Bestimmung der Chlordioxid-Konzentration:
Die Chlordioxid-Lösung wurde in einem üblichen Chlordioxid-Generator durch Mischen einer 2 bis 40 %-igen Natriumchlorit-Lösung mit einer 1 bis 60 %-igen Lösung der sauren Komponente bei Raumtemperatur hergestellt. In einem charakteristischen Beispiel betrug die Konzentration der Natriumchlorit-Lösung 7,5 % und die Konzentration der sauren Komponente zwischen 15 und 25 %. Als saure Komponente wurde hierbei ein Gemisch aus 2-Phosphonobutan-1,2,4-tricarbonsäure und Citronensäure im Molverhältnis 1 : 1 eingesetzt. Die Reaktionstemperatur kann im Bereich von 0 °C bis 60 °C variiert werden, um die Gleichgewichtseinstellung des Chlordioxids zu verändern. Um homogene und kontinuierliche Chlordioxid-Konzentrationen zu erzielen, ist eine mittlere Verweilzeit von 30 sec bis 20 min notwendig, bevor es zur Anwendung kommt. Die Wirksamkeit des Chlordioxids und der Sequestrierung treten momentan nach dem Durchlaufen der Reaktionszone auf.

Als üblicher Generator wurde eine BelloZon CD 035 Erzeugungsanlage der Fa. ProMinent, D-6900 Heidelberg benutzt. Es können aber für kleinere Anwendungszwecke einfache Spender oder mechanische Apparate benutzt werden, die die beiden Komponenten einfach vermischen und somit zur Reaktion bringen (z.B. Instrumentendesinfektion in Krankenhäusern). Für großflächigere Einsätze eignen sich Anlagen, die die Chemikalien konzentriert dosieren und mit Hilfe einer Verdünnungspumpe ein Entstehen explosiver Chlordioxidkonzentrationen in der Anwendungslösung verhindern.

Die Bestimmung der Chlordioxid-Konzentration erfolgte photometrisch mit N,N-Diethyl-1,4-phenylendiamin als Redoxindikator oder on-line amperometrisch. Dabei wurde die Konzentration mit 0,1 bis 20 ppm, vorzugsweise mit 0,5 bis 5 ppm festgestellt. Für übliche Anwendungen sind in der Regel 2 ppm ausreichend. Eine Steuerung der Chlordioxid-Konzentration kann durch das Verhältnis der Konzentration der sauren Komponente zu Natriumchlorit erfolgen.

Die erzielten Ausbeuten an Chlordioxid betrugen bis zu 97 % der Theorie.

Beispiele:

Für die nachstehenden Beispiele wurde als Reaktor der vorstehend erwähnte Bellozon CD 035-Generator eingesetzt. Im Reaktor wurde jeweils eine 7,5 gew.-%ige wäßrige Natriumchloritlösung vorgelegt und mit der jeweiligen "sauren Komponente" beziehungsweise Säure (siehe die einzelnen Beispiele) innig vermischt.

Die Reaktion verläuft nach der folgenden allgemeinen Gleichung:

5 Mol $NaClO_2$ + 4 Mol Säure --> 4 Mol $ClO_2$ + 5 Mol Salz + 2 Mol $H_2O$.

Hiernach ist für einen stöchiometrischem Umsatz ein Zusatz von 4 Mol Säure beziehungsweise saurer Komponente zu 5 Mol Natriumchlorit ausreichend. In den nachstehenden Beispielen wurde die jeweilige saure Komponente jedoch mit einem Überschuß von ca. 10 Mol - bezogen auf das vorstehende Molverhältnis - eingesetzt.

Zur Bestimmung der Chlordioxid-Ausbeute beim Einsatz verschiedener saurer Komponenten wurde der Gehalt der Lösung an Chlordioxid (in ppm) mit der maximal theoretisch erzielbaren Konzentration beim HCl-Chlorit-Verfahren verglichen. Der Umsatz beträgt in diesem Falle 8,42 ppm Chlordioxid, entsprechend 100 % Umsatz, bezogen auf die nachfolgenden Beispiele.

1. Es wurden eingesetzt:
5 Mol $NaClO_2$ = 452,2 g,
14,2 Mol "saure Komponente", bestehend aus:
6,12 Mol Citronensäure = 1175,6 g
2,89 Mol 2-Phosphonobutan-1,2,4-tricarbonsäure (Bayhibit$^R$AM, Fa. Bayer AG) = 779,3 g
2,29 Mol 1-Hydroxyethan-1,1-diphosphonsäure (Turpinal$^R$SL, Fa. Henkel KGaA) = 470,9 g
2,90 Mol Polyacrylsäure (Sokalan$^R$DCS, Fa. BASF AG) = 391,8 g,
Ausbeute: 3,88 Mol $ClO_2$ (= 261,7 g), entsprechend 97 % der Theorie.

Das nachfolgende Beispiel wurden in analoger Weise durchgeführt:
2) Säure: Gemisch aus 2-Phosphonobutan-1,2,4-tricarbonsäure und Citronensäure im Molverhältnis 1 : 1, Ausbeute: 8,16 ppm $ClO_2$ (= 97 % der Theorie).

**Patentansprüche**

**1.** Verfahren zur Desinfektion von harten Oberflächen unter Wasserhärte-stabilisierten und nicht korrosiven Bedingungen mit wäßrigen Lösungen von Chlordioxid, hergestellt in Lösung durch Mischen einer Lösung aus Natriumchlorit und einer sauren Komponente in einem Generator,
dadurch gekennzeichnet, daß
a) die Konzentration der Natriumchlorit-Lösung 2 bis 40 Gew.-% beträgt,
b) als saure Komponente, Wasserhärtestabilisator und Korrosionsinhibitor ein stickstofffreies Sequestrierungsmittel oder Komplexierungsmittel, ausgewählt aus Phosphonsäuren, Polyphosphorsäure, Acrylsäure, Methacrylsäure, Polyacrylsäure oder Polymethacrylsäure, oder Gemische derartiger Sequestrierungsmittel und Komplexierungsmittel verwendet werden,
c) wobei das Sequestrierungsmittel und/oder Komplexierungsmittel, dessen wäßrige 1 %-ige Lösung einen pH-Wert von < 7 aufweist, in einer Konzentration von 1 bis 60 Gew.-% eingesetzt wird,
d) zusätzlich zu den Sequestrierungsmitteln und/oder Komplexierungsmitteln Carbonsäuren und/oder Hydroxycarbonsäuren, ausgewählt aus Weinsäure, Äpfelsäure, Oxalsäure, Maleinsäure, Malonsäure, Bernsteinsäure, Adipinsäure, Glycolsäure, Milchsäure, Gluconsäure oder Citronensäure, eingesetzt werden.
e) wobei das Mischungsverhältnis (bezogen auf Molverhältnisse) von Sequestrierungs- und/oder Komplexierungsmittel zu Carbonsäure oder Hydroxycarbonsäure im Bereich von 10 : 1 bis 1 : 10 liegt,
f) und die Chlordioxid-Konzentration zwischen 0,1 und 500 ppm beträgt.

**2.** Verfahren nach Anspruch 1, dadurch gekennzeichnet, daß ein Sequestrierungsmittel und/oder Komplexierungsmittel eingesetzt wird, dessen wäßrige 1 %-ige Lösung einen pH-Wert von < 3 aufweist und die Chlordioxid-Konzentration zwischen 0,1 und 100 ppm beträgt.

**3.** Verfahren nach Ansprüchen 1 und 2, dadurch gekennzeichnet, daß 1-Hydroxyethan-1,1-diphosphonsäure und/oder 2-Phosphonobutan-1,2,4-tricarbonsäure als Sequestrierungs- und/oder Komplexierungsmittel eingesetzt werden.

**4.** Verfahren nach Ansprüchen 1 bis 3, dadurch gekennzeichnet, daß die Konzentration des Sequestrierungsmittels und/oder Komplexierungsmittel 15 bis 25 Gew.-% beträgt.

**5.** Verfahren nach Ansprüchen 1 bis 4, dadurch gekennzeichnet, daß die Lösung mehr als die theoretische Menge an saurer Komponente, bezogen auf Natriumchlorit, enthält.

**6.** Verfahren nach Ansprüchen 1 bis 5, dadurch gekennzeichnet, daß die Chlordioxid-Konzentration 0,1 bis 20 ppm, insbesondere 0,5 bis 5 ppm, beträgt.

**7.** Verfahren nach Ansprüchen 1 bis 6, dadurch gekennzeichnet, daß als Carbonsäure und/oder Hydroxycarbonsäure Milchsäure oder Citronensäure eingesetzt werden.

**8.** Verfahren nach Ansprüchen 1 bis 7, dadurch gekennzeichnet, daß zusätzlich zu den Sequestrierungsmitteln und/oder Komplexierungsmitteln und den Carbonsäuren und/oder Hydroxycarbonsäuren Lewissäuren, vorzugsweise Eisen- oder Aluminium-sulfate, -nitrate oder -chloride, eingesetzt werden.

## Claims

**1.** A process for disinfecting hard surfaces under water-hardness-stabilized and non-corrosive conditions with aqueous solutions of chlorine dioxide produced in solution by mixing of a solution of sodium chlorite and an acidic component in a generator, characterized in that

a) the concentration of the sodium chlorite solution is between 2 and 40% by weight,

b) a nitrogen-free sequestering agent or complexing agent selected from phosphonic acids, polyphosphoric acid, acrylic acid, methacrylic acid, polyacrylic acid or polymethacrylic acid or mixtures of such sequestering agents and complexing agents is/are used as the acidic component, hardness stabilizer and corrosion inhibitor,

c) the sequestering agent and/or complexing agent, of which a 1% aqueous solution has a pH value of $<7$, being used in a concentration of 1 to 60% by weight,

d) carboxylic acids and/or hydroxycarboxylic acids selected from tartaric acid, malic acid, oxalic acid, maleic acid, malonic acid, succinic acid, adipic acid, glycolic acid, lactic acid, gluconic acid or citric acid are used in addition to the sequestering agents and/or complexing agents,

e) the mixing ratio (based on molar ratios) of sequestering and/or complexing agent to carboxylic acid or hydroxycarboxylic acid being from 10:1 to 1:10,

f) and the chlorine dioxide concentration is between 0.1 and 500 ppm.

**2.** A process as claimed in claim 1, characterized in that a sequestering agent and/or complexing agent having a pH value of $<3$ in the form of a 1% aqueous solution is used and the chlorine dioxide concentration is between 0.1 to 100 ppm.

**3.** A process as claimed in claims 1 and 2, characterized in that 1-hydroxyethane-1,1-diphosphonic acid and/or 2-phosphonobutane-1,2,4-tricarboxylic acid is/are used as the sequestering and/or complexing agent(s).

**4.** A process as claimed in claims 1 to 3, characterized in that the concentration of the sequestering agent and/or complexing agent is between 15 and 25% by weight.

**5.** A process as claimed in claims 1 to 4, characterized in that the solution contains more than the theoretical quantity of acidic component, based on sodium chlorite.

**6.** A process as claimed in claims 1 to 5, characterized in that the concentration of chlorine dioxide is between 0.1 and 20 ppm and, more particularly, between 0.5 and 5 ppm.

**7.** A process as claimed in claims 1 to 6, characterized in that lactic acid or citric acid is used as the carboxylic acid and/or hydroxycarboxylic acid.

**8.** A process as claimed in claims 1 to 6, characterized in that Lewis acids, preferably iron or aluminium sulfates, nitrates or chlorides, are used in addition to the sequestering agents and/or complexing agents and the carboxylic acids and/or hydroxycarboxylic acids.

## Revendications

**1.** Procédé de désinfection de surfaces dures dans des conditions de stabilité vis-à-vis de la dureté de l'eau et non corrosives, avec des solutions aqueuses de dioxyde de chlore, produit en solution par mélange d'une solution à base de chlorite de sodium et d'un composant acide dans un générateur, caractérisé en ce que :

a) la concentration de la solution de chlorite de sodium s'élève à 2 jusqu'à 40 % en poids,
b) comme composant acide, comme agent stabilisant de la dureté de l'eau et comme inhibiteur de corrosion, on utilise un agent séquestrant ou complexant, dépourvu d'azote, choisi parmi les acides phosphoniques, l'acide polyphosphorique, l'acide acrylique, l'acide méthacrylique, l'acide polyacrylique ou l'acide polyméthacrylique ou bien des mélanges d'agents séquestrants et d'agents complexants de ce type,
c) l'agent séquestrant et/ou l'agent complexant, dont la solution aqueuse à 1 % possède une valeur de pH de < 7, est mis en oeuvre à une concentration de 1 à 60 % en poids,
d) en supplément aux agents séquestrants et/ou aux agents complexants, on met en jeu des acides carboxyliques et/ou des acides carboxyliques hydroxylés choisis parmi l'acide tartrique, l'acide malique, l'acide oxalique, l'acide maléique, l'acide malonique, l'acide succinique, l'acide adipique, l'acide glycolique, l'acide lactique, l'acide gluconique ou l'acide citrique,
e) le rapport de mélange (rapporté aux rapports molaires) d'agent séquestrant et/ou d'agent complexant aux acides carboxyliques ou aux acides carboxyliques hydroxylés se situe dans la zone de 10:1 à 1:10,
f) et la concentration en dioxyde de chlore s'élève entre 0,1 et 500 ppm.

**2.** Procédé selon la revendication 1, caractérisé en ce que l'on met en oeuvre un agent séquestrant et/ou un agent complexant dont la solution aqueuse à 1 % possède une valeur de pH de < 3 et que la concentration en dioxyde de chlore s'élève entre 0,1 et 100 ppm.

**3.** Procédé selon les revendications 1 et 2, caractérisé en ce que l'acide 1-hydroxyéthane-1,1-diphosphonique et/ou l'acide 2-phosphonobutane-1,2,4-tricarboxylique sont mis en oeuvre comme agent séquestrant et/ou agent complexant.

**4.** Procédé selon les revendications 1 à 3, caractérisé en ce que la concentration en agent séquestrant et/ou en agent complexant, s'élève de 15 à 25 % en poids.

**5.** Procédé selon les revendications 1 à 4, caractérisé en ce que la solution renferme plus que la quantité théorique en composant acide, rapportée au chlorite de sodium.

**6.** Procédé selon les revendications 1 à 5, caractérisé en ce que la concentration en dioxyde de chlore s'élève de 0,1 à 20 ppm, en particulier de 0,5 à 5 ppm.

**7.** Procédé selon les revendications 1 à 6, caractérisé en ce que l'on met en oeuvre comme acide carboxylique et/ou comme acide carboxylique hydroxylé de l'acide lactique ou de l'acide citrique.

**8.** Procédé selon les revendications 1 à 7, caractérisé en ce qu'en supplément aux agents séquestrants et/ou agents complexants et aux acides carboxyliques et/ou aux acides carboxyliques hydroxylés, on met en oeuvre des acides de Lewis, de préférence des sulfates des nitrates ou des chlorures de fer ou d'aluminium.